(19) Europäisches Patentamt
European Patent Office
Office européen des brevets

(11) Veröffentlichungsnummer: **0 072 970**
**A1**

# (12) EUROPÄISCHE PATENTANMELDUNG

(21) Anmeldenummer: 82107264.2

(22) Anmeldetag: 11.08.82

(51) Int. Cl.³: C 07 C 131/00
A 01 N 37/50

(30) Priorität: 22.08.81 DE 3133359

(43) Veröffentlichungstag der Anmeldung: 02.03.83 Patentblatt 83/9

(84) Benannte Vertragsstaaten: AT BE CH DE FR GB IT LI NL SE

(71) Anmelder: BAYER AG
Zentralbereich Patente, Marken und Lizenzen
D-5090 Leverkusen 1, Bayerwerk(DE)

(72) Erfinder: Brandes, Wilhelm, Dr.
Eichendorffstrasse 3
D-5653 Leichlingen(DE)

(72) Erfinder: Daum, Werner, Dr.
Baerenstrasse 18
D-4150 Krefeld(DE)

(54) **Substituierte Anilinomethylen-oxime, Verfahren zu ihrer Herstellung sowie ihre Verwendung als Pflanzenschutzmittel.**

(57) Substituierte Anilinomethylene-oxime der Formel (I)

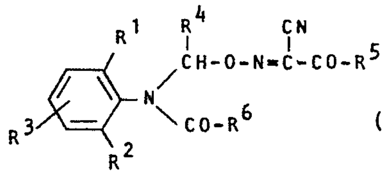

(I),

in welcher

$R^1$ Alkyl, Alkoxy oder Halogen,
$R^2$ Wasserstoff, Alkyl oder Halogen,
$R^3$ Wasserstoff, Alkyl oder Halogen,
$R^4$ Wasserstoff oder Methyl,
$R_5$ Alkoxy, Amino oder $-NH-CO-NH-R^7$ bedeutet, wobei $R^7$ für Wasserstoff oder gegebenenfalls substituiertes Alkyl, Cycloalkyl, Alkoxyalkyl steht und
$R^6$ Alkoxy, Alkoxycarbonyl, gegebenenfalls substituiertes Alkyl, Alkenyl, Alkinyl, Cycloalkyl, Aryl, Aralkyl, Aryloxyalkyl, Arylthioalkyl oder einen heterocyclischen Rest oder eine Heterocyclylalkylgruppe bedeutet.

Verfahren zu ihrer Herstellung, sowie ihre Verwendung als Pflanzenschutzmittel.

EP 0 072 970 A1

0072970

BAYER AKTIENGESELLSCHAFT
Zentralbereich
Patente, Marken und Lizenzen
5090 Leverkusen, Bayerwerk
Eck/bo/c
Ib

# Substituierte Anilinomethylen-oxime, Verfahren zu ihrer Herstellung sowie ihre Verwendung als Pflanzenschutzmittel

Die vorliegende Erfindung betrifft neue substituierte Anilinomethylen-oxime, Verfahren zu ihrer Herstellung sowie ihre Verwendung als Pflanzenschutzmittel.

Wie bereits lange bekannt ist, werden im Pflanzenschutz Fungizide in der Landwirtschaft und im Gartenbau, insbesondere das Zink-ethylen-1,2-bis-dithiocarbamidat und das N-Trichlormethylthio-tetrahydrophthalimid verwendet; die genannten Verbindungen besitzen unter den Handelsprodukten eine große Bedeutung (vgl. R. Wegler, "Chemie der Pflanzenschutz- und Schädlingsbekämpfungsmittel", Band 2, Seiten 65 und 108, Berlin/Heidelberg/New York (1970)). Die Wirkung bei niedrigen Aufwandkonzentrationen ist jedoch nicht immer ganz befriedigend.

Weiterhin ist bekannt, daß einige Isonitroso-cyanacetamid-Derivate (DE-OS 2 312 956, US-PS 3 919 284,



BAD ORIGINAL

3 957 847 und 4 188 401) und Alkoxycarbonylethyl-N-haloacetylaniline (DE-OS 2 350 944) fungizide Eigenschaften besitzen. Auch hier ist die Wirksamkeit bei niedrigen Aufwandmengen nicht ganz befriedigend und bei höheren Konzentrationen werden oft Pflanzenschäden gesehen.

Es wurden neue substituierte Anilinomethylen-oxime der Formel (I)

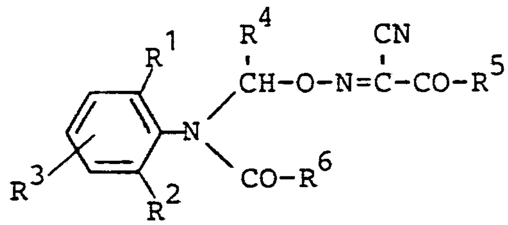

(I),

gefunden,

in welcher

$R^1$ Alkyl, Alkoxy oder Halogen,
$R^2$ Wasserstoff, Alkyl oder Halogen,
$R^3$ Wasserstoff, Alkyl oder Halogen,
$R^4$ Wasserstoff oder Methyl,
$R^5$ Alkoxy, Amino oder $-NH-CO-NH-R^7$ bedeutet, wobei $R^7$ für Wasserstoff oder gegebenenfalls substituiertes Alkyl, Cycloalkyl, Alkoxyalkyl steht und
$R^6$ Alkoxy, Alkoxycarbonyl, gegebenenfalls substituiertes Alkyl, Alkenyl, Alkinyl, Cycloalkyl, Aryl, Aralkyl, Aryloxyalkyl, Arylthioalkyl oder einen heterocyclischen Rest oder eine Heterocyclylalkylgruppe

bedeutet.

Die erfindungsgemäßen Anilinomethylen-oxime der allgemeinen Formel (I) können in verschiedenen geometrischen Strukturen vorliegen. Formel (I) umfaßt alle möglichen geometrischen Strukturen.

Man erhält die substituierten Anilinomethylen-oxime der Formel (I)

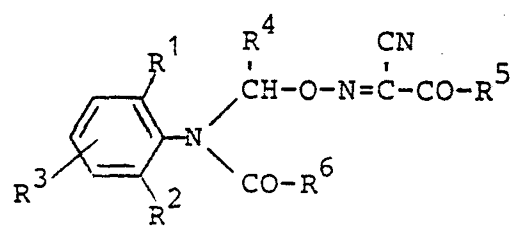

(I),

in welcher

$R^1$ Alkyl, Alkoxy oder Halogen,
$R^2$ Wasserstoff, Alkyl oder Halogen,
$R^3$ Wasserstoff, Alkyl oder Halogen,
$R^4$ Wasserstoff oder Methyl,
$R^5$ Alkoxy, Amino oder $-NH-CO-NH-R^7$ bedeutet, wobei $R^7$ für Wasserstoff oder gegebenenfalls substituiertes Alkyl, Cycloalkyl, Alkoxyalkyl steht und

$R^6$ Alkoxy, Alkoxycarbonyl, gegebenenfalls substituiertes Alkyl, Alkenyl, Alkinyl, Cycloalkyl, Aryl, Aralkyl, Aryloxyalkyl, Arylthioalkyl oder einen heterocyclischen Rest oder eine Heterocyclylalkylgruppe

bedeutet,

wenn man

a) ein N-Halogenalkylanilin der Formel (II)

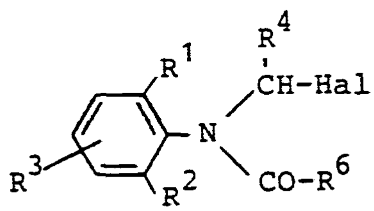

(II),

in welcher

$R^1$, $R^2$, $R^3$, $R^4$ und $R^6$ die oben angegebenen Bedeutungen besitzen und

Hal für eine Abgangsgruppe wie Chlor, Brom oder Iod steht,

mit einem 2-Cyan-2-oximinoessigsäure-Derivat der Formel (III)

$$HON=\overset{CN}{\overset{|}{C}}-CO-R^5$$ (III),

in welcher

$R^5$ die oben genannte Bedeutung hat,

in Gegenwart eines Säurebinders umsetzt, oder daß man

b) erfindungsgemäße Verbindungen der Formel (IV)

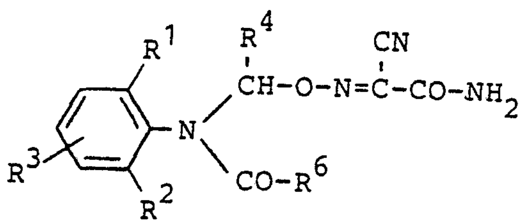

(IV),

in welcher

$R^1$, $R^2$, $R^3$, $R^4$ und $R^6$ die oben angegebenen Bedeutungen haben,

in Gegenwart einer Base mit einem Isocyanat der Formel (V)

$$OCN-R^7 \quad (V),$$

in welcher

$R^7$ die oben angegebene Bedeutung hat, aber nicht für Wasserstoff steht,

umsetzt.

Die neuen Anilinomethylen-oxime der Formel (I) weisen starke fungizide Eigenschaften auf. Sie sind protektiv, curativ und eradikativ anwendbar. Außerdem haben sie systemische und/oder locosystemische Eigenschaften. Überraschenderweise zeigen sie eine bessere Pflanzenverträglichkeit als die bekannten Isonitrosocyanacetamid-Derivate. Gegenüber den Dithiocarbamidaten und dem N-Trichlormethylthio-tetrahydrophthalimid besitzen sie den Vorteil curativer und eradikativer Wirkung.

Die erfindungsgemäßen Verbindungen stellen schon wegen der vielen Möglichkeiten ihrer überlegenen biologischen Anwendung eine wertvolle Bereicherung der Technik dar. Ein weiterer wesentlicher Gesichtspunkt dieser Erfindung ist, daß neue Wirkstoffe mit für die Praxis wertvollen Eigenschaften zu einer Zeit zur Verfügung gestellt werden, da durch Resistenzerscheinungen ältere Wirkstoffe für den Markt ausfallen.

Es besteht daher heute und in absehbarer Zeit ein ausgesprochener Bedarf nach neuen Fungiziden.

Von den erfindungsgemäßen Anilinomethylen-oximen der Formel (I) sind diejenigen bevorzugt, bei denen

$R^1$ für Alkyl und Alkoxy mit jeweils 1 bis 4 C-Atomen oder Halogen steht,

$R^2$ für Wasserstoff, Alkyl mit 1 bis 4 C-Atomen oder Halogen steht,

$R^3$ für Wasserstoff, Alkyl mit 1 bis 4 C-Atomen oder Halogen steht,

$R^4$ für Wasserstoff oder Methyl steht,

$R^5$ für Alkoxy mit 1 bis 4 C-Atomen, Amino oder $-NH-CO-NHR^7$ steht, wobei $R^7$ für Wasserstoff oder gegebenenfalls substituiertes Alkyl mit 1 bis 5 C-Atomen oder Alkoxyalkyl mit 2 bis 5 C-Atomen steht und

$R^6$ für Alkoxy mit 1 bis 4 C-Atomen, Alkoxycarbonyl mit 1 bis 4 C-Atomen, gegebenenfalls substituiertes Alkyl mit 1 bis 6 C-Atomen, gegebenenfalls substituiertes Alkenyl mit 2 bis 5 C-Atomen, Alkinyl mit 2 bis 4 C-Atomen, Cycloalkyl mit 3 bis 7 C-Atomen, gegebenenfalls substituiertes Phenyl, Benzyl, Phenylethyl, Phenyloxymethyl, Phenylthiomethyl, Phenylthioethyl und einen gegebenenfalls substituierten, gesättigten oder ungesättigten heterocyclischen Rest oder Heterocyclylmethylgruppe steht, wobei die Heterocyclen als Heteroatome ein Sauerstoff- oder Schwefelatom, bis zu drei Stickstoffatome oder bis zu zwei Stickstoffatome und ein Sauerstoff- oder ein Schwefelatom enthalten können.

Als Halogen sei beispielsweise Chlor und Brom genannt.

Als Substituenten des Restes $R^7$ kommen bevorzugt Cyangruppen und Alkoxycarbonylgruppen mit 1 bis 4 C-Atomen in Frage.

Steht $R^6$ für Alkyl, kann er bevorzugt Substituenten wie Alkoxy, Alkylthio, Alkylsulfonyloxy, Alkylsulfoxy, Alkylsulfonyl, Alkoxyalkylthio, Alkanoyloxy und Alkoxyalkoxy mit jeweils 1 bis 4 C-Atomen in jedem Alkylteil, Propargyloxy, die Cyano- oder Rhodanogruppe und Halogen wie Fluor, Chlor und Brom tragen. Steht $R^6$ für Alkenyl, Alkinyl oder Cycloalkyl, kann er bevorzugt Substituenten wie Halogen, beispielsweise Fluor, Chlor und Brom tragen. Steht $R^6$ für Aryl und Aralkyl so kann er bevorzugt bis zu drei Substituenten, beispielsweise Halogen wie Fluor, Chlor und Brom, Trifluormethyl, Cyan, Nitro, Alkyl und Alkyl-(thio)oxy mit jeweils 1 bis 4 C-Atomen, und Methylendioxy, tragen. Steht $R^6$ für Aryloxymethyl oder Arylthioalkyl kann er bevorzugt Substituenten wie Halogen, beispielsweise Chlor, Alkyl, beispielsweise Methyl und Trifluormethyl tragen. Steht $R^6$ für einen heterocyclischen Rest oder eine Heterocyclylalkylgruppe, kann er bevorzugt Substituenten, beispielsweise Alkylgruppen, Methyl und Trifluormethylgruppen tragen.

Neben den bei den Herstellungsbeispielen genannten erfindungsgemäßen Verbindungen seien beispielhaft folgende weitere erfindungsgemäße Verbindungen der allgemeinen Formel (I) genannt:

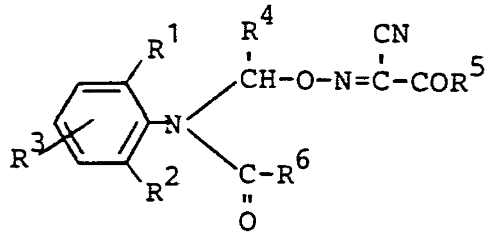

I

| $R^1$ | $R^2$ | $R^3$ | $R^4$ | $R^5$ | $R^6$ | $R^7$ |
|---|---|---|---|---|---|---|
| $CH_3$ | $CH_3$ | H | H | $NHR^7$ | $CH_2Cl$ | $CO-NH-C_6H_{11}$ |
| $CH_3$ | $CH_3$ | H | H | $NHR^7$ | $CHCl_2$ | $CO-NH-C_6H_{11}$ |
| $CH_3$ | $CH_3$ | H | H | $NHR^7$ | $CCl_3$ | $CO-NH-C_6H_{11}$ |
| $CH_3$ | $CH_3$ | H | H | $NHR^7$ | $CH_2CN$ | $CO-NH-C_6H_{11}$ |

| $R^1$ | $R^2$ | $R^3$ | $R^4$ | $R^5$ | $R^6$ | $R^7$ |
|---|---|---|---|---|---|---|
| $CH_3$ | $CH_3$ | H | H | $NHR^7$ | $CH_2SCN$ | $CO-NH-C_6H_{11}$ |
| $CH_3$ | $CH_3$ | H | H | $NHR^7$ | $CH_2OCH_3$ | $CO-NH-C_6H_{11}$ |
| $CH_3$ | $CH_3$ | H | H | $NHR^7$ | $CH_2OC_2H_5$ | $CO-NH-C_6H_{11}$ |
| $CH_3$ | $CH_3$ | H | H | $NHR^7$ | $OCH_3$ | $CO-NH-C_6H_{11}$ |
| $CH_3$ | $CH_3$ | H | H | $NHR^7$ | $OC_2H_5$ | $CO-NH-C_6H_{11}$ |
| $CH_3$ | $CH_3$ | H | H | $NHR^7$ | $OCH(CH_3)_2$ | $CO-NH-C_6H_{11}$ |
| $CH_3$ | $CH_3$ | H | H | $NHR^7$ | 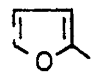 | $CO-NH-C_6H_{11}$ |
| $CH_3$ | $CH_3$ | H | H | $NHR^7$ | $CH_2OCH_2$ | $CO-NH-(CH_2)_5-COOCH_3$ |
| $CH_3$ | $CH_3$ | H | H | $NH_2$ | $CO-OC_2H_5$ | - |
| $C_2H_5$ | $CH_3$ | H | H | $NHR^7$ | $CH_2Cl$ | $CO-NH-C_4H_9$ |
| $C_2H_5$ | $CH_3$ | H | H | $NH_2$ | $CH_2Cl$ | - |
| $C_2H_5$ | $CH_3$ | H | H | $NH_2$ | $CH_2CN$ | - |
| $C_2H_5$ | $CH_3$ | H | H | $NHR^7$ | $CH_2Cl$ | $CO-NH_2$ |
| $C_2H_5$ | $CH_3$ | H | H | $NH_2$ | $CH_2Br$ | - |
| $C_2H_5$ | $CH_3$ | H | H | $NHR^7$ | $CH_2Br$ | $CO-NH_2$ |
| $C_2H_5$ | $CH_3$ | H | H | $NH_2$ | $CH_2-O-CH_3$ | - |
| $C_2H_5$ | $C_2H_5$ | H | H | $NHR^7$ | $CH_2Cl$ | $CO-NH-CH_3$ |
| $C_2H_5$ | $C_2H_5$ | H | H | $NHR^7$ | $CH_2Cl$ | $CO-NH_2$ |
| $C_2H_5$ | $C_2H_5$ | H | H | $NH_2$ | $CH_2Cl$ | - |
| $C_2H_5$ | $C_2H_5$ | H | H | $NHR^7$ | $CH_2Br$ | $CO-NH_2$ |
| $C_2H_5$ | $C_2H_5$ | H | H | $NH_2$ | $CH_2Br$ | - |
| $C_2H_5$ | $C_2H_5$ | H | H | $NH_2$ | $CH_2CN$ | - |
| $C_2H_5$ | $C_2H_5$ | H | H | $NH_2$ | $CH_2SCN$ | - |
| $C_2H_5$ | $C_2H_5$ | H | H | $NH_2$ | $CH_2O-CH_3$ | - |
| $C_2H_5$ | $C_2H_5$ | H | $CH_3$ | $NH_2$ | $CH_2Cl$ | - |
| $C_2H_5$ | $CH_3$ | $CH_3$ | H | $NH_2$ | $CH_2OCH_3$ | - |
| $i-C_3H_7$ | $CH_3$ | H | H | $NH_2$ | $CH_2Cl$ | - |
| $i-C_3H_7$ | $CH_3$ | H | H | $NHR^7$ | $CH_2Cl$ | $CO-NH-C_2H_5$ |
| $i-C_3H_7$ | $CH_3$ | H | H | $NH_2$ | $CH_2Br$ | - |
| $i-C_3H_7$ | $CH_3$ | H | H | $NHR^7$ | $CH_2Br$ | $CO-NH-(CH_2)_5-CN$ |
| $t-C_4H_9$ | $CH_3$ | H | H | $NH_2$ | $CH_2Cl$ | - |
| $t-C_4H_9$ | $CH_3$ | H | H | $NHR^7$ | $CH_2Cl$ | $CO-NH_2$ |
| $t-C_4H_9$ | $CH_3$ | H | H | $NH_2$ | $CH_2Br$ | - |
| $t-C_4H_9$ | $CH_3$ | H | H | $NHR^7$ | $CH_2Br$ | $CO-NH-CH_3$ |

Verwendet man bei Verfahrensvariante a) beispielsweise N-Chlormethyl-N-(2-ethoxyacetyl)-2,6-dimethylanilin und 2-Cyan-2-oximinoessigsäuremethylester als Ausgangsstoffe und N-Ethyl-N,N-diisopropylamin als Säurebinder, so kann der Reaktionsverlauf durch das folgende Formelschema wiedergegeben werden:

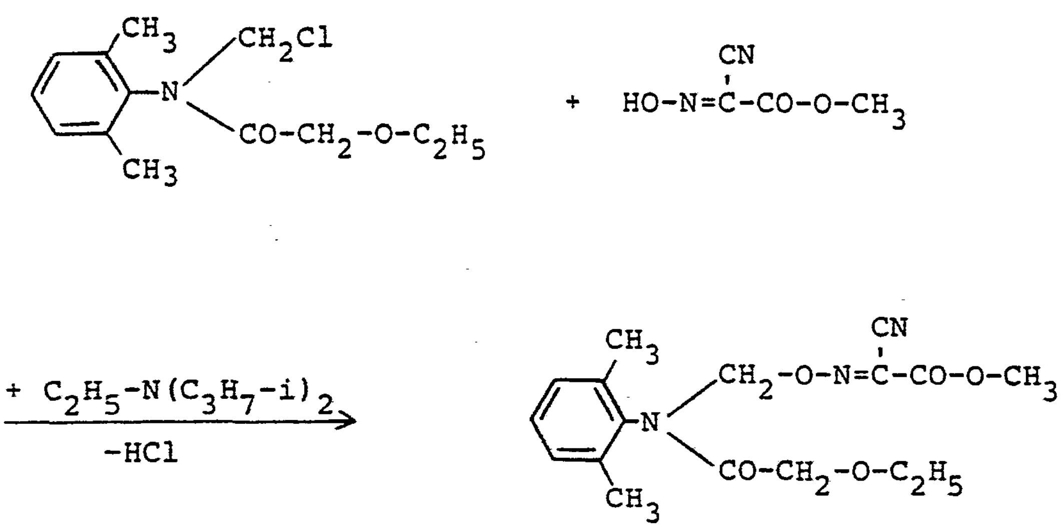


Verwendet man bei Verfahrensvariante b) beispielsweise 2-Cyan-2-O-(N-(2,6-dimethylphenyl)-N-(furan-2-oyl)-aminomethyl)-oximinoacetamid, Natriumhydrid und n-Propylisocyanat als Ausgangsstoffe, so kann der Reaktionsverlauf durch das folgende Formelschema wiedergegeben werden:

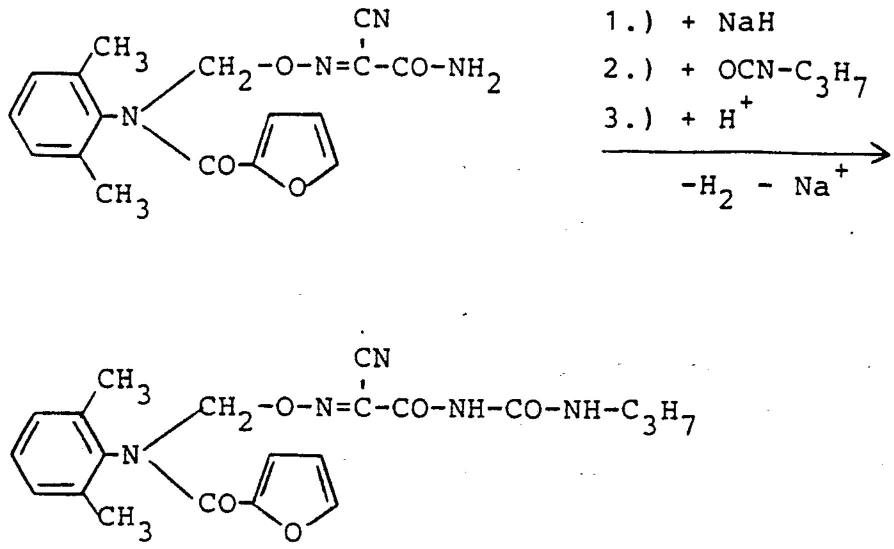


Die als Ausgangsstoffe eingesetzten N-Halogenalkylaniline sind durch Formel (II) allgemein definiert. In dieser Formel haben die Reste $R^1$ bis $R^4$ und $R^6$ vorzugsweise die Bedeutung, die bereits im Zusammenhang mit Formel (I) vorzugsweise genannt wurde.

Erfindungsgemäß verwendbare N-Halogenalkyl-aniline der Formel (II) sind bekannt oder können nach prinzipiell bekannten Verfahren, beispielsweise durch Umsetzung von geeigneten Anilinen mit Aldehyden zu den Schiffschen Basen und anschließender Umsetzung mit Säurehalogeniden hergestellt werden (US-PS 3 630 716, 3 637 847; DOS 1 542 950, 1 793 811, 2 854 598, 2 854 599, 2 854 600, EU-PA 2 90 11 und CA 64, 10760 g). Eine zwischenzeitliche Isolierung der N-Halogenalkyl-aniline der Formel (II) ist nicht unbedingt erforderlich.

Als Beispiele seien genannt:
N-Bromacetyl-N-brommethyl-2,6-dimethylanilin; N-Acetyl-, N-Chloracetyl-, N-Dichloracetyl-, N-Trichloracetyl-, N-2-Cyanacetyl-, N-2-Methoxyacetyl-, N-2-Ethoxyacetyl-,

N-2-Propoxyacetyl-, N-2-Isopropoxyacetyl-, N-Methoxyethyl-sulfonylacetyl-, N-Ethoxy-ethylsulfenylacetyl-, N-Propionyl-, N-2-Chlorpropionyl-, N-Butanoyl-, N-Methoxycarbonylacyl-, N-Ethoxycarbonylacyl-, N-Isopropoxycarbonylacyl-, N-Butoxycarbonylacyl-, N-Methoxycarbonyl-, N-Ethoxycarbonyl-, N-Isopropoxycarbonyl-N-chlormethyl-2,6-dimethylanilin; N-Furanoyl-N-(1-chlorethyl)-2-chlor-6-methylanilin.

Weiterhin werden für die erfindungsgemäßen Umsetzungen nach Verfahrensvariante a) 2-Cyan-2-oximino-essigsäure-Derivate der allgemeinen Formel (III) benötigt. Diese Verbindungen sind teilweise bekannt (Chem. Ber. 42, 736 - 741 (1909); 54, 1334 (1921); US-PS 4 188 401).

So erhält man beispielsweise oximierte Harnstoffe der Formel

$$HO-N=\overset{\displaystyle CN}{\overset{|}{C}}-CO-NH-CO-NH-R^7 \qquad (IIIa),$$

in welcher

$R^7$ mit Ausnahme von Wasserstoff die bei Formel (I) angegebene Bedeutung hat,

wenn man
Isocyanate der Formel (V)

$$R^7-NCO \qquad (V),$$

in welcher

$R^7$ mit Ausnahme von Wasserstoff die bei Formel (I) angegebene Bedeutung hat,

in einer ersten Stufe mit Ammoniak zu N-subsituiertem Harnstoff umsetzt und in einer zweiten Stufe diesen mit Cyanessigsäure in Gegenwart von Acetanhydrid zum entsprechenden 1-substituierten 3-(2-cyanacetyl)-harnstoff umsetzt und diesen mit salpetriger Säure zum entsprechenden oximierten Harnstoff umsetzt.

Im erfindungsgemäßen Verfahren a) einsetzbare oximierte Harnstoffe bzw. Cyanessigsäure-Derivate der Formel (III) sind beispielsweise:
2-Cyan-2-oximinoessigsäure-methyl-, -ethyl-, -propyl-, -isopropyl- und -sek.-butyl-ester; 2-Cyan-2-oximinoacetamid; $N^1$-(2-Cyan-2-oximinoacetyl)-harnstoff; 1-(2-Cyan-2-oximinoacetyl)-3-methyl-, -ethyl-, -propyl-, -isopropyl-, -butyl-, -isobutyl-, -amyl-, -ω-cyanethyl-, -ω-cyanpentyl-, -ω-ethoxycarbonyl-methyl-, -ω-methoxycarbonylmethyl-, -ω-methoxycarbonylpentyl-, -methoxymethyl-, -3-methoxypropyl-, -cyclopropyl-, -cyclopropylmethyl-, -cyclopentyl- und -cyclohexyl-harnstoff.

Als Verdünnungsmittel können für das Verfahren a) alle gegenüber den Reaktionspartnern inerten organischen Lösungsmittel in Frage kommen, vorzugsweise polare Lösungsmittel, beispielsweise Acetonitril, Aceton, Chloroform, Benzonitril, Dimethylacetamid, Dimethylformamid, Dimethylsulfoxid, Chlorbenzol, Essigsäureethylester, Dioxan, Methylethylketon, Methylenchlorid und Tetrahydrofuran, und unpolare Lösungsmittel, beispielsweise Toluol.

Die Umsetzung kann gegebenenfalls auch in Mischungen aus Wasser und einem wassermischbaren organischen Lösungsmittel durchgeführt werden oder in heterogenen Systemen, bestehend aus Wasser und einem mit Wasser nicht mischbaren oder nur teilweise mischbaren Lösungsmittel.

Als Säurebinder werden organische Basen, vorzugsweise tertiäre Amine, beispielsweise Chinolin, Dimethylbenzylamin, N,N-Dimethylanilin, Ethyldicyclohexylamin, Ethyldiisopropylamin, Picolin, Pyridin und Triethylamin verwendet oder die 2-Cyan-2-oximinoessigsäurederivate werden in Form ihrer Alkali- oder Erdalkalisalze eingesetzt.

Die erfindungsgemäße Umsetzung wird bei einer Temperatur von -50 bis 80°C, bevorzugt von -30 bis 40°C ausgeführt. Bei Verwendung oder Mitverwendung von Wasser als Verdünnungsmittel wird bei einer Temperatur vom Erstarrungspunkt der wäßrigen Lösung bis etwa +60°C, vorzugsweise von 0 bis +40°C umgesetzt.

Das erfindungsgemäße Verfahren kann beispielsweise wie folgt ausgeführt werden:

Ein 2-Cyan-2-oximinoessigsäure-Derivat der Formel (III), in einem Verdünnungsmittel dispergiert oder gelöst, wird vorgelegt und mit einer molaren Menge eines tertiären Amins versetzt, wobei Salzbildung eintreten kann. Wird ein Alkali- oder Erdalkalisalz des 2-Cyan-2-oximinoessigsäure-Derivats eingesetzt, kann dieses in einem inerten Lösungsmittel vorgelegt werden. Dazu wird das N-Halogenalkylanilin, vorzugsweise gelöst in einem Verdünnungsmittel, gegeben. Nach Beendigung der Umsetzung werden die Anilinomethylenoxime auf übliche Art isoliert und gegebenenfalls gereinigt.

In einer besonderen Verfahrensweise setzt man dem Umsetzungsgemisch vor Beginn der Reaktion eine kleine Menge eines Iodids zu.

Im erfindungsgemäßen Verfahren b) einsetzbare Anilinomethylen-oxime der Formel (IV) sind erfindungsgemäße Verbindungen. In Formel (IV) haben die Reste $R^1$ bis $R^4$ und $R^6$ vorzugsweise die Bedeutungen, die im Zusammenhang mit Formel (I) vorzugsweise genannt wurden.

Bei der Formel (V) der im erfindungsgemäßen Verfahren einsetzbaren Isocyanate hat $R^7$ vorzugsweise die Bedeutung mit Ausnahme von Wasserstoff, die im Zusammenhang mit Formel (I) vorzugsweise genannt wurde.

Die Isocyanate der Formel (V) sind bekannte Verbindungen. Sie können auf übliche Art, beispielsweise durch Umsetzung von primären Aminen mit Phosgen, hergestellt werden.

Im einzelnen seien folgende Verbindungen genannt: Methyl-, Ethyl-, Propyl-, Isopropyl-, Butyl-, sek.-Butyl-, ω-Cyanethyl, 1-Cyan-1-methyl-ethyl-, ω-Cyan-propyl-, ω-Cyanpentyl-, Methoxycarbonyl-methyl-, Ethoxycarbonyl-methyl, Propoxycarbonyl-ethyl-, 1-Methoxycarbonyl-1-methyl-ethyl-, 1-Ethoxycarbonyl-1-methyl-ethyl-, Ethoxycarbonyl-1-ethyl-ethyl-, Methoxycarbonyl-1-ethyl-ethyl-, Methoxycarbonyl-propyl-, Methoxycarbonyl-pentyl-, Isopropoxycarbonyl-pentyl-, Cyclohexyl-isocyanat.

Als Verdünnungsmittel können indifferente wasserfreie Iösungsmittel wie Ether, beispielsweise Diisopropylether, Dioxan oder Tetrahydrofuran verwendet werden.

Die Umsetzung wird bei einer Temperatur von -20 bis 80°C, vorzugsweise 20 bis 60°C, durchgeführt.

Als Basen können Alkoholate, beispielsweise Alkalialkoholate wie Natriummethoxid und Kalium-tert.-butoxid und Metallhydride, beispielsweise Alkalihydride wie Natriumhydrid und Kaliumhydrid eingesetzt werden.

Das erfindungsgemäße Verfahren b) wird wie folgt durchgeführt:
Das 2-Cyan-2-(N-acylanilino-alkoxyimino)-acetamid-Derivat der Formel (IV) wird in einem Verdünnungsmittel mit einer vorstehend genannten Base zum entsprechenden Anion des Amids umgesetzt und dieses mit dem Isocyanat der Formel (V) in dem angegebenen Temperaturbereich umgesetzt. Nach beendeter Reaktion wird in der Kälte mit einer organischen Carbonsäure wie Essigsäure schwach angesäuert.

Je nach Umsetzungsbedingungen fallen die erfindungsgemäßen Wirkstoffe kristallin aus oder sie bleiben im organischen Lösungsmittel gelöst und können dann nach Auswaschen der Lösung mit Wasser durch vorsichtiges Einengen der Lösung oder durch Zugabe wenig polarer organischer Lösungsmittel, wie Cyclohexan, Dibutylether, Butylacetat oder Tetrachlorkohlenstoff, abgeschieden werden. Gegebenenfalls müssen mit Wasser mischbare Lösungsmittel nach der Reaktion durch Abdampfen im Vakuum entfernt werden.

Sind die erfindungsgemäßen Verbindungen in einem mit Wasser mischbaren Lösungsmittel gelöst, so können sie beispielsweise durch Zugabe von Wasser ausgefällt werden. Soweit es die besonderen Bedingungen der Aufarbeitungsprozesse erlauben, werden die Lösungen der erfindungsgemäßen Wirkstoffe, bzw. die noch Lösungsmittel-feuchten Suspensionen der Wirkstoffe schwach sauer eingestellt.

Die erfindungsgemäßen Verbindungen können sich bei höherer Temperatur zersetzen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.



BAD ORIGINAL

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Oomyceten, z.B. gegen den Erreger der Kraut- und Braunfäule der Kartoffel und Tomate (Phytophthora infestans), eingesetzt werden.

Sie zeigen eine hohe kurative und protektive Wirksamkeit. Daneben sind gute Wirkungen gegen Rostpilze festzustellen.

Die erfindungsgemäßen Wirkstoffe weisen nicht nur die guten Eigenschaften hervorragender Handelspräparate auf, sondern besitzen darüber hinaus noch erhebliche Vorteile. Diese liegen in erster Linie in der Fähigkeit der erfindungsgemäßen Stoffe, in die Pflanze einzudringen. Sie können aufgenommen werden von der Saatgutoberfläche, von den Wurzeln und auch von oberirdischen Pflanzenorganen nach äußerlichen Applikationen. Auch besitzen sie die vorteilhafte Fähigkeit, locosystemisch zur Wirkung zu kommen, d.h. eine Tiefenwirkung im Pflanzengewebe auszuüben und dabei pilzliche Krankheitserreger zu eliminieren, die bereits in das Gewebe der Wirtspflanze eingedrungen sind.

Die erfindungsgemäßen Verbindungen können zur Verbreiterung ihres fungiziden Wirkungsspektrums, zur Erhöhung ihrer Wirksamkeit und zur Verzögerung der Resistenzbildung mit bekannten fungiziden Wirkstoffen zusammen appliziert werden.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage:

z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Zusätzlich zu den obigen Formulierungsmöglichkeiten ist zu bemerken, daß die erfindungsgemäßen Stoffe zusammen mit Saccharose, Dextrose, Dextrinen, mit wasserfreiem Calciumsulfat oder Calciumsulfat-hemihydrat, sowie mit Carbonsäuren, wie z.B. Fumarsäure oder 4-Hydroxylbenzoesäure, oder auch mit schwach sauren Ionenaustauschern formuliert werden können.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B.

Eisenoxid, Titanoxid, Ferrocyanblau und organische Farbstoffe wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele:

Beispiel 1

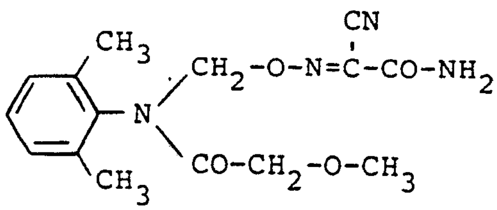

14,2 g (0,125 M) 2-Cyan-2-oximinoacetamid und 250 ml Acetonitril werden vorgelegt. Unter Kühlung mit Eis werden 12,6 g Triethylamin zugetropft und nach einer halben Stunde eine Lösung von 30 g (0,124 M) N-Chlormethyl-N-methoxyacetyl-2,6-dimethylanilin in 400 ml Diisopropylether und 400 ml Toluol zugegeben. Der Ansatz wird 90 min lang auf 40°C gehalten. Die Reaktionsmischung wird im Vakuum eingeengt, der Rückstand mit Petrolether und mit Wasser gewaschen. Man erhält 27 g 2-Cyan-2-(O-(N-methoxyacetyl-N-2,6-dimethylphenyl-aminometh)-oximino)-acetamid, Fp. 173 - 174°.

Das Produkt schmilzt nach Umkristallisieren aus Acetonitril bei 174 - 175,5°.

Auf analoge Weise werden hergestellt:

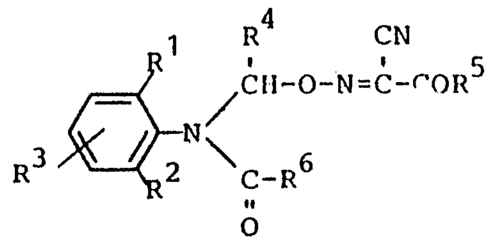

| Bsp.-Nr. | $R^1$ | $R^2$ | $R^3$ | $R^4$ | $R^5$ | $R^6$ | Physik. Konst. |
|---|---|---|---|---|---|---|---|
| 2 | $CH_3$ | $CH_3$ | H | H | $NHCONHR^7$ $R^7=H$ | $CH_2OCH_3$ | 156 - 160°C |
| 3 | $CH_3$ | $CH_3$ | H | H | $NHCONHR^7$ $R^7=C_2H_5$ | $CH_2OCH_3$ | 113,5°C |
| 4 | $CH_3$ | $CH_3$ | H | H | $NHCONHR^7$ $R^7=(CH_2)_5CN$ | $CH_2OCH_3$ | 136 - 138°C |
| 5 | $CH_3$ | $CH_3$ | H | H | $NH_2$ | 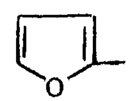 | 184°C |
| 6 | $CH_3$ | $CH_3$ | H | H | $NH_2$ | $CH_2Cl$ | 180°C |
| 7 | $CH_3$ | $CH_3$ | H | H | $NHCONHR^7$ $R^7=C_2H_5$ | $CH_2Cl$ | 135°C |

(Fortsetzung)

| Bsp.-Nr. | $R^1$ | $R^2$ | $R^3$ | $R^4$ | $R^5$ | $R^6$ | Physik. Konst. |
|---|---|---|---|---|---|---|---|
| 8 | $CH_3$ | $CH_3$ | H | H | $OCH_3$ | $CH_2-O-CH_3$ | 105°C |
| 9 | $CH_3$ | $CH_3$ | H | H | $NH_2$ | $OCH_3$ | 187,5°C |
| 10 | $CH_3$ | $CH_3$ | H | H | $NH_2$ | $CH_2SCN$ | IR ($CHCl_3$) 2160 $cm^{-1}$ |
| 11 | $CH_3$ | $CH_3$ | H | $CH_3$ | $NH_2$ | $CH_2Cl$ | 153°C |
| 13 | $CH_3$ | $CH_3$ | H | H | $NH_2$ | $CH_2-S-CH_3$ | 133°C |
| 14 | $CH_3$ | $CH_3$ | H | H | $NH_2$ | $CHCl-CH_3$ | 150°C |

Beispiel 12

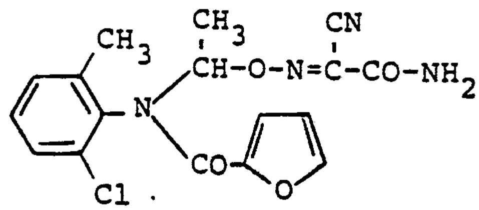

45,7 ml Acetaldehyd werden in 10 min bei 3°C zu einer Mischung aus 73,6 g 2-Chlor-6-methylanilin, 403 ml Toluol, 0,5 ml konz. Schwefelsäure und 86 g Zeolith getropft. Die Mischung wird 24 h bei 20°C gerührt. Es wird filtriert und im Vakuum weitgehend eingeengt. Der Abdampfrückstand wird in 370 ml Toluol gelöst und bei -30°C zu einer Lösung von 33 g Furan-2-carbonsäurechlorid in 200 ml Toluol getropft. Nach 3 h wird diese Reaktionsmischung zu einer auf -30°C gekühlten Mischung aus 28,2 g 2-Cyan-2-oximino-acetamid, 200 ml Acetonitril und 26 g Triethylamin gegeben. Das Reaktionsgemisch bleibt über Nacht bei Zimmertemperatur stehen. Dann wird filtriert. Das Filtrat wird im Vakuum eingedampft und der Rückstand mit Butylacetat behandelt. 2-Cyan-2-(O-(1-(N-furan-2-oyl-N-2-chlor-6-methylphenyl-amino)-ethyl)-oximino)-acetamid kristallisiert aus; Fp. 153°C;

Cl gef. 9,25 - 9,33 %

ber. 9,46 %.

Beispiel A

Phytophthora-Test (Tomate) /protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1, 5, 6.

Patentansprüche

1. Substituierte Anilinomethylen-oxime der Formel I

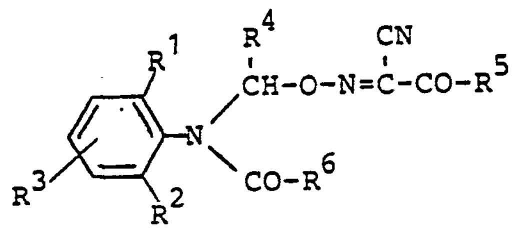

(I),

in welcher

$R^1$ Alkyl, Alkoxy oder Halogen,

$R^2$ Wasserstoff, Alkyl oder Halogen,

$R^3$ Wasserstoff, Alkyl oder Halogen,

$R^4$ Wasserstoff oder Methyl,

$R^5$ Alkoxy, Amino oder $-NH-CO-NH-R^7$ bedeutet, wobei $R^7$ für Wasserstoff oder gegebenenfalls substituiertes Alkyl, Cycloalkyl, Alkoxyalkyl steht und

$R^6$ Alkoxy, Alkoxycarbonyl, gegebenenfalls substituiertes Alkyl, Alkenyl, Alkinyl, Cycloalkyl, Aryl, Aralkyl, Aryloxyalkyl, Arylthioalkyl oder einen heterocyclischen Rest oder eine Heterocyclylalkylgruppe

bedeutet.

2. Verfahren zur Herstellung von substituierten Anilinomethylenoximen der Formel (I)

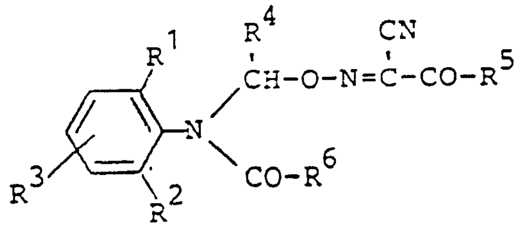

(I),

in welcher

$R^1$ Alkyl, Alkoxy oder Halogen,
$R^2$ Wasserstoff, Alkyl oder Halogen,
$R^3$ Wasserstoff, Alkyl oder Halogen,
$R^4$ Wasserstoff oder Methyl,
$R^5$ Alkoxy, Amino oder -NH-CO-NH-$R^7$ bedeutet, wobei $R^7$ für Wasserstoff oder gegebenenfalls substituiertes Alkyl, Cycloalkyl, Alkoxyalkyl steht und

$R^6$ Alkoxy, Alkoxycarbonyl, gegebenenfalls substituiertes Alkyl, Alkenyl, Alkinyl, Cycloalkyl, Aryl, Aralkyl, Aryloxyalkyl, Arylthioalkyl oder einen heterocyclischen Rest oder eine Heterocyclylalkylgruppe

bedeuten, dadurch gekennzeichnet, daß man

a) ein N-Halogenalkylanilin der Formel (II)

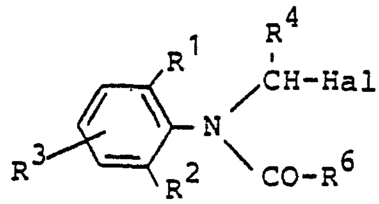

(II),

in welcher

$R^1$, $R^2$, $R^3$, $R^4$ und $R^6$ die oben angegebenen Bedeutungen besitzen und

Hal für eine Abgangsgruppe wie Chlor, Brom oder Iod steht,

mit einem 2-Cyan-2-oximinoessigsäure-Derivat der Formel (III)

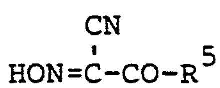

(III),

in welcher

$R^5$ die obengenannte Bedeutung hat,

in Gegenwart eines Säurebinders umsetzt, oder daß man

b) erfindungsgemäße Verbindungen der Formel (IV)

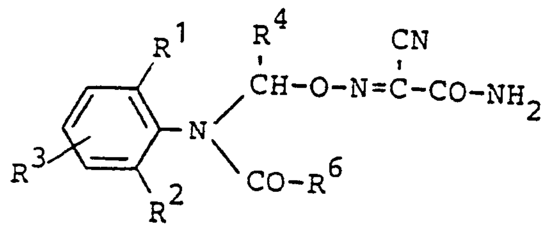

(IV),

in welcher

$R^1$, $R^2$, $R^3$, $R^4$ und $R^6$ die oben angegebenen Bedeutungen haben,

in Gegenwart einer Base mit einem Isocyanat der Formel (V)

$$OCN-R^7 \qquad (V),$$

in welcher

$R^7$ die oben angegebene Bedeutung hat, aber nicht für Wasserstoff steht,

umsetzt.

3. Substituierte Anilinomethylen-oxime der Formel (I), in welcher

$R^1$ für Alkyl und Alkoxy mit jeweils 1 bis 4 C-Atomen oder Halogen steht,

$R^2$ für Wasserstoff, Alkyl mit 1 bis 4 C-Atomen oder Halogen steht,

$R^3$ für Wasserstoff, Alkyl mit 1 bis 4 C-Atomen oder Halogen steht,

$R^4$ für Wasserstoff oder Methyl steht,

$R^5$ für Alkoxy mit 1 bis 4 C-Atomen, Amino oder $-NH-CO-NHR^7$ steht, wobei $R^7$ für Wasserstoff oder gegebenenfalls substituiertes Alkyl mit 1 bis 5 C-Atomen, Cycloalkyl mit 3 bis 6 C-atomen oder Alkoxyalkyl mit 2 bis 5 C-Atomen steht und

$R^6$ für Alkoxy mit 1 bis 4 C-Atomen, Alkoxycarbonyl mit 1 bis 4 C-Atomen, gegebenenfalls substituiertes Alkyl mit 1 bis 6 C-Atomen, gegebenenfalls substituiertes Alkenyl mit 2 bis 5 C-Atomen, Alkinyl mit 2 bis 4 C-Atomen, Cycloalkyl mit 3 bis 7 C-Atomen, gegebenenfalls substituiertes Phenyl, Benzyl, Phenylethyl, Phenyloxymethyl, Phenylthiomethyl, Phenylthioethyl und einen gegebenenfalls substituierten, gesättigten oder ungesättigten heterocyclischen Rest oder eine Heterocyclylmethylgruppe steht, wobei die Heterocyclen als Heteroatome ein Sauerstoff- oder ein Schwefelatom, bis zu drei Stickstoffatome oder bis zu zwei Stickstoffatome und ein Sauerstoff- oder ein Schwefelatom enthalten können.

4. 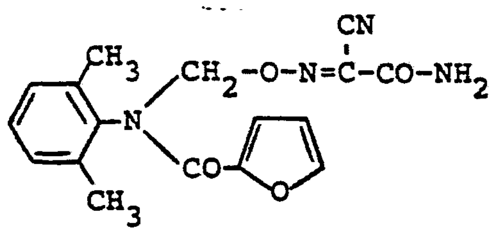

5\. 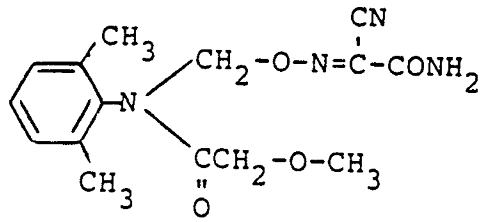

6\. 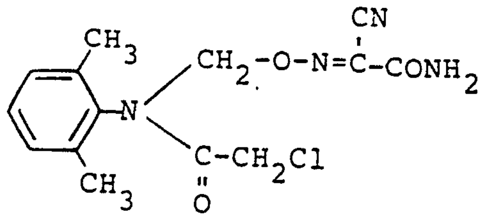

7\. Fungizides Mittel, gekennzeichnet durch einen Gehalt an mindestens einem substituierten Anilinomethylen-oxim gemäß Anspruch 1.

8\. Verfahren zur Bekämpfung von Pilzen, dadurch gekennzeichnet, daß man substituierte Anilinomethylen-oxime gemäß Anspruch 1 auf Pilze oder ihren Lebensraum einwirken läßt.

9\. Verwendung von substituierten Anilinomethylenoximen gemäß Anspruch 1 zur Bekämpfung von Pilzen.

10\. Verfahren zur Herstellung von fungiziden Mitteln, dadurch gekennzeichnet, daß man substituierte Anilinomethylen-oxime gemäß Anspruch 1 mit Streckmitteln und/oder oberflächenaktiven Mitteln vermischt.

Europäisches Patentamt

# EUROPÄISCHER RECHERCHENBERICHT

0072970

Nummer der Anmeldung

EP 82 10 7264

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | GB-A-2 029 223 (CIBA-GEIGY)<br>* Seiten 1,2 *<br>--- | 1,7 | C 07 C 131/00<br>A 01 N 37/50 |
| Y | CH-A- 558 336 (CIBA-GEIGY)<br>* Spalte 1 *<br>----- | 1,7 | |

| RECHERCHIERTE SACHGEBIETE (Int. Cl. ³) |
|---|
| C 07 C 131/00<br>A 01 N 37/00 |

Der vorliegende Recherchenbericht wurde für alle Patentansprüche erstellt

| Recherchenort | Abschlußdatum der Recherche | Prüfer |
|---|---|---|
| DEN HAAG | 06-10-1982 | VERHULST W. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
&: Mitglied der gleichen Patentfamilie, übereinstimmendes Dokument

EPA Form 1503 03 82